# EUROPÄISCHE PATENTSCHRIFT

(11) **EP 2 470 353 B1**
(45) Veröffentlichungstag und Bekanntmachung des Hinweises auf die Patenterteilung: **01.10.2014**
(21) Anmeldenummer: 10750049.8
(22) Anmeldetag: 24.08.2010
(51) Int. Cl.: B30B 11/00, A61F 13/20

(54) **PRESSE ZUR HERSTELLUNG EINES TAMPONS**
PRESS FOR PRODUCING A TAMPON
PRESSE POUR RÉALISER UN TAMPON

(30) Priorität: 24.08.2009 AT 13312009
(43) Veröffentlichungstag der Anmeldung: 04.07.2012
(73) Patentinhaber: Ruggli Projects AG, 6332 Hagendorn (CH)
(72) Erfinder: GRABER, Heinz, CH-8962 Bergdietikon (CH)
(74) Vertreter: Burger, Hannes
(86) Internationale Anmeldenummer: PCT/EP2010/005177
(87) Internationale Veröffentlichungsnummer: WO 2011/023364

(56) Entgegenhaltungen:
- EP-A1- 1 108 408
- WO-A1-00/53141
- WO-A1-01/01905
- DE-A1- 3 606 150
- DE-C1- 19 825 877
- US-A- 3 422 496

## Beschreibung

Die Erfindung betrifft eine Presse zur Herstellung eines Tampons durch radiales Pressen eines aus einem absorbierenden Material hergestellten Tamponrohlings mittels Pressbacken, welche um einen Pressbereich angeordnet sind.

Weiters betrifft die Erfindung ein Verfahren zur Herstellung eines Tampons durch radiales Pressen eines aus einem absorbierenden Material hergestellten Tamponrohlings mittels Pressbacken, welche um einen Pressbereich einer Presse angeordnet sind.

Tampons sind an sich bekannte Hygieneartikel und können aus Wattebändern durch Rollen und anschließendem Verdichten in einer Pressvorrichtung hergestellt werden, wobei sich üblicherweise eine herstellungsbedingt kreiszylinderförmige Grundform des Tampons ergibt. Aus dem Stand der Technik sind seit langem viele unterschiedliche Tampons bekannt, deren Oberflächeneigenschaften physikalisch und/oder chemisch verändert werden, um sowohl ästhetische als auch funktionelle Vorteile zu verleihen. In diesem Zusammenhang sind Pressvorrichtungen bekannt geworden, um Tampons mit uneinheitlicher Oberflächentopografie - vor allem in Form von Längsrillen, gegebenenfalls unterschiedlicher Tiefe, in der Oberfläche des Tampons - herzustellen. Eine derartige Presse der eingangs genannten Art ist beispielsweise aus der EP 0639 363 bekannt geworden. Die bekannte Presse weist einen Pressbereich mit radial zu einem Tampon verschiebbaren Pressbacken auf, welche je ein Eindringungssegment zum Eindringen in das absorbierende Material aufweisen. Nachteilig bei den bekannten Pressen ist, dass der Tampon üblicherweise relativ unkontrolliert in den Pressbereich eingeführt wird, wodurch es zu einer nicht lagerichtigen Positionierung des Tampons in dem Pressbereich kommen kann. Unterschiedliche Positionierungen von Tampons entlang der Achse des Pressbereichs haben zur Folge, dass in die Oberfläche eingeprägte Muster an unterschiedlichen Stellen der Tampons aufscheinen. Ein einheitliches Erscheinungsbild der mit der Presse hergestellten Tampons ist somit bei den herkömmlichen Pressen nicht gewährleistet. Durch eine Verschiebung des Tamponrohlings in radialer Richtung kann es zu einer inhomogenen Verdichtung kommen, was zu einer Beeinträchtigung der Qualität des fertigen Tampons führen kann.

Es ist daher eine Aufgabe der Erfindung, die oben genannten Nachteile zu überwinden.

Diese Aufgabe wird mit einer Presse der eingangs genannten Art erfindungsgemäß dadurch gelöst, dass die Presse Führungsklingen zur Zentrierung des Tamponrohlings in radialer und/oder axialer Richtung in dem Pressbereich aufweist.

Durch die Verwendung von Führungsklingen kann eine exakte Positionierung eines Tampons in dem Pressbereich und somit eine wesentliche Verbesserung der Qualität des gepressten Tampons erzielt werden.

Eine besonders gute Führung des Tamponrohlings lässt sich dadurch erzielen, dass die Führungsklingen in Richtung des Pressbereichs betrachtet radial um diesen angeordnet sind.

Eine besonders vorteilhafte Ausführungsform der Erfindung, besteht darin, dass jede Führungsklinge zwischen zwei benachbarten Pressbacken angeordnet ist. Hierdurch lässt sich eine sehr platzsparende Anordnung realisieren, die sich darüber hinaus dadurch auszeichnet, dass auf diese Weise Einschränkung hinsichtlich der Bewegung der Pressbacken vermieden werden können. Die Führungsklingen sind ortsfest in der Presse montiert. Durch die ortsfeste Montage der Führungsklingen, können die Pressbacken, während des Pressens des Tamponrohlings auf einander zu bewegt werden, ohne dass die Führungsklingen diese Bewegungen mitvollziehen und den eigentlichen Pressvorgang behindern.

Eine exakte Positionierung des Tampons in axialer Richtung kann dadurch erreicht werden, dass die Führungsklingen in Einführrichtung des Tamponrohlings in den Pressbereich aufeinander zulaufend angeordnet und/oder ausgebildet sind.

Gemäß einer vorteilhaften Variante der Erfindung, die sich durch einen besonders einfachen Aufbau auszeichnet, können die Führungsklingen unbeweglich in der Presse montiert sein.

Um eine gute Führung des Tampons während des Einschubvorganges in den Pressbereich zu gewährleisten, können zumindest drei Führungsklingen vorgesehen sein.

Um ein Einführen des Tamponrohlings zwischen die Führungsklingen zu erleichtern, können die Führungsklingen in einem zur Aufnahme des Tamponrohlings vorgesehenen Aufnahmebereich weiter von einander beabstandet sein als in einem von dem Aufnahmebereich entfernten Abschnitt.

Eine weitere Möglichkeit, eine exakte Positionierung des Tamponrohlings in axialer Richtung in dem Pressbereich zu erzielen, besteht darin, dass zumindest eine Führungsklinge an ihrem dem Pressbereich zugewandten Ende einen Anschlag für den Tamponrohling aufweist.

Ein gutes Führungsverhalten zur Positionierung des Tamponrohlings lässt sich dadurch erzielen, dass die Führungsklingen federelastisch ausgebildet sind.

Ganz allgemein gesprochen, können die Führungsklingen plattenförmig ausgebildet sein, wobei je eine in das Innere des Pressbereichs weisende Kante der plattenförmigen Führungsklingen zur Führung des Tamponrohlings vorgesehen ist.

Gemäß einer Ausführungsform der Erfindung, die sich durch einen besonders günstigen Aufbau auszeichnet, der auch gewährleistet, dass es zu keiner Behinderung der Pressbacken während des Pressvorganges kommt, können die Führungsklingen zumindest abschnittsweise an einem ringförmigen Befestigungselement angeordnet und von diesem an ihrer Position gehalten sein. Alternativ hierzu können die Führungsklingen an einer Ausstossdüse der Presse angeordnet sein.

Die oben genannte Aufgabe kann auch mit einem Verfahren der eingangs genannten Art erfindungsgemäß dadurch gelöst werden, dass der Tamponrohling während des Einführens in den Pressbereich mittels Führungsklingen in radialer und/oder axialer Richtung in dem Pressbereich zentriert wird.

Die Erfindung samt weiteren Vorteilen wird im Folgenden anhand einiger nicht einschränkender Ausführungsbeispiele näher erläutert, welche in den Zeichnungen dargestellt sind. In diesen zeigen schematisch:
- Fig. 1: eine Draufsicht auf einen Pressbereich einer erfindungsgemäßen Presse;
- Fig. 2: einen Schnitt entlang der Linie II-II in Fig. 1 einer ersten Variante der Erfindung;
- Fig. 3: einen Schnitt entlang der Linie II-II in Fig. 1 einer zweiten Variante der Erfindung;
- Fig. 4: einen Schnitt entlang der Linie II-II in Fig. 1 einer dritten Variante der Erfindung;
- Fig. 5: eine Seitenansicht einer Variante der Erfindung, bei welcher die Führungsklingen an einer Ausstossdüse der Presse angeordnet sind;
- Fig. 6: eine Draufsicht auf die Variante aus Fig. 5;
- Fig. 7: einen Schnitt entlang der Linie VII-VII in Fig. 5;
- Fig. 8: eine perspektivische Ansicht der Variante aus Fig. 5;
- Fig. 9: den Pressbereich aus Fig. 1 mit geöffneten Pressbacken;
- Fig. 10: den Pressbereich aus Fig. 1 während eines Pressvorganges mit aufeinander zu bewegten, geschlossenen Pressbacken und
- Fig. 11: den Pressbereich aus Fig. 1 während eines Lüftvorganges zur Entfernung des gepressten Tamponvorformlings aus dem Pressbereich.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

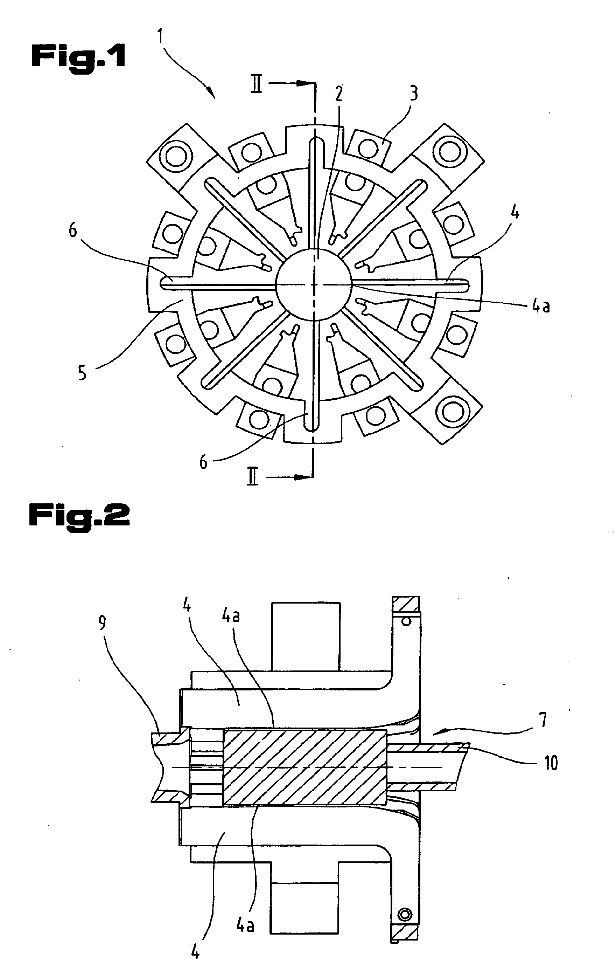

Gemäß Fig. 1 weist eine erfindungsgemäße Presse 1 radial um einen Pressbereich zum Verdichten eines Tamponrohlings 2 angeordnete Pressbacken 3 auf. Der Tamponrohling 2 kann beispielsweise durch Rollen oder Falten eines Vliesstücks aus einem absorbierenden Material, beispielsweise Watte, geformt werden. Dieser Tamponrohling 2 wird dann in den zwischen den Pressbacken 3 liegenden Pressbereich der Presse 1 eingeführt und mittels der Pressbacken 3 radial verdichtet.

Um ein axiales und/oder radiales Zentrieren des zu verdichtenden Tamponrohlings 2 in der Presse 1 zu ermöglichen, sind Führungsklingen 4 vorgesehen, durch welche der Tamponrohling 2 an eine genau definierte Position innerhalb des zwischen den Pressbacken 3 Pressbereichs geführt wird.

Der Begriff "axial" bezieht sich hierbei auf die Längsachse des zwischen den Pressbacken 3 liegenden Pressbereichs der Presse 1. Die Bezeichnung "radial" bezieht sich auf eine normal zur Längsachse des Pressbereichs verlaufende Richtung.

Die radiale Zentrierung des Tamponrohlings 2 in dem Pressbereich hat zur Folge, dass die Längsachse des Pressbereichs mit der Längsachse des Tamponrohlings 2 im Wesentlichen zusammen fällt, während die axiale Zentrierung die Positionierung des Tamponrohlings 2 entlang der Längsachse des Pressbereichs betrifft.

Wie aus Fig. 1 ersichtlich ist jede Führungsklinge 4 zwischen zwei benachbarten Pressbacken 3 angeordnet ist. Die Führungsklingen 4 sind ortsfest montiert.

Die Führungsklingen 4 können plattenförmig ausgebildet sein, wobei je eine dem Inneren der Presse 1 zugewandte Kante 4a der plattenförmigen Führungsklingen 4 zur Führung des Tamponrohlings 2 vorgesehen ist. Mit dem Begriff "plattenförmig" wird hierbei ganz allgemein ein Körper mit ebenen Deckflächen bezeichnet dessen Dicke deutlich kleiner ist als seine Länge und Breite. Grundsätzlich können die Führungsklingen 4 auch anders als plattenförmig ausgestaltet sein, allerdings bietet sich die plattenförmige Ausbildung aufgrund ihrer einfachen Form und Montage an. Darüber hinaus können die Führungsklingen 4 federelastisch aber auch starr ausgebildet sein. Die Kanten 4a der Führungsklingen 4 müssen nicht eben bzw. geradlinig ausgebildet sein, sondern können auch beispielsweise gekrümmt oder gewellt ausgebildet sein. Grundsätzlich können die Kanten 4a jedoch auch jede andere geeignete Form annehmen.

Weiters können die Führungsklingen 4 je mit einem Abschnitt an einem Befestigungsring 5 angebracht sein, wie aus Fig. 1 ersichtlich ist. Die Pressbacken 3 können in Richtung der Einführbewegung des Tamponrohlings 2 betrachtet nach dem Befestigungsring 5 angeordnet sein, sodass es durch den Befestigungsring 5 zu keiner Beeinträchtigung der Bewegung der Pressbacken 3 kommt. Der Befestigungsring 5 selbst kann mit entsprechenden Befestigungsmitteln an der Presse 1 befestigt sein, beispielsweise mittels einer Schraubverbindung. Natürlich ist auch jede andere Befestigungsart des Befestigungsringes 5 an der Presse 1 möglich, beispielsweise durch Verschweißen, Verkleben, Verriegeln etc.

Wie in Fig. 1 dargestellt, kann der Befestigungsring 5 Aufnahmen 6 für die Führungsklingen 4 aufweisen. Die Führungsklingen 4 können durch Verkleben, Verschweißen, Löten, Klemmen, Verschrauben etc. mit dem Befestigungsring 5 verbunden werden. Die Art der Verbindung der Führungsklingen 4 mit dem Befestigungsring 5 hängt nicht zuletzt von der Art der verwendeten Materialien ab. So können der Befestigungsring 5 bzw. die Führungsklingen 4 beispielsweise aus Metall oder einem Kunststoff hergestellt sein. Prinzipiell wäre es auch möglich, die Führungsklingen 4 und den Befestigungsring 5 einstückig miteinander auszubilden.

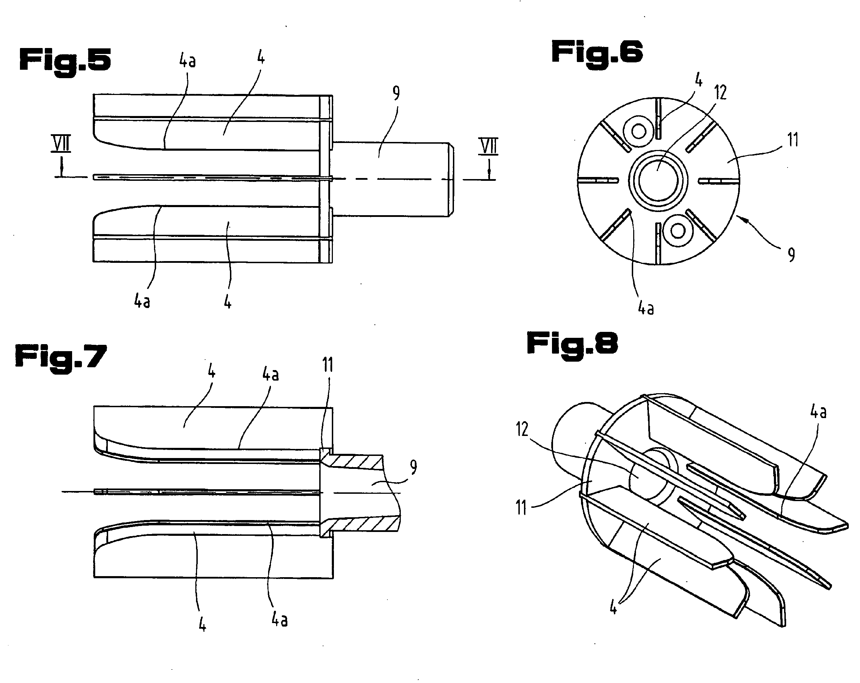

Zur Befestigung der Führungsklingen 4 muss jedoch nicht unbedingt ein Befestigungsring 5 vorgesehen sein. So können die Führungsklingen 4 auch, wie in den Figuren 5 bis 8 näher dargestellt, an einer Ausstossdüse der Presse 1 befestigt sein, durch welche der mit den Pressbacken 3 zu einem Tamponvorformling 2' verdichtete Tamponrohling 2 aus der Presse 1 befördert wird. In den Figuren 2 und 5 bis 8 ist die Ausstossdüse mit dem Bezugszeichen 9 versehen.

Mittels der Führungsklingen 4 wird der Tamponrohling 2 während des Einführens so weit abgebremst, dass er an der vorgesehenen Stelle des Pressbereichs zum Stillstand kommt und exakt positioniert ist. Die erfindungsgemäße Lösung erlaubt es, auch bei hohen Einführgeschwindigkeiten des Tamponrohlings 2 diesen so abzubremsen, dass er innerhalb der Presse 1 genau an der vorgesehenen Position zum Stillstand kommt. Insbesondere lässt sich durch die erfindungsgemäße Lösung ein durch die Massenträgheit des Tamponrohlings 2 bedingtes Zurückfedern des Tamponrohlings 2 von einer den Pressbereich begrenzenden Rückwand bzw. von der Ausstossdüse 9 der Presse 1 verhindern.

Wie aus Fig. 1 weiters ersichtlich ist, können die Führungsklingen 4 radial um den Pressbereich angeordnet sein. Anstelle der dargestellten acht Führungsklingen 4 kann jedoch eine beliebige andere Zahl von Führungsklingen 4 vorgesehen sein. Vorteilhafterweise werden jedoch mindestens drei Führungsklingen 4 verwendet, um eine ausreichend gute Führung des Tamponrohlings 2 zu gewährleisten.

Aus Fig. 2 ist ersichtlich, dass in einem zur Aufnahme des Tamponrohlings 2 vorgesehenen Aufnahmebereich 7 die Führungsklingen 4 weiter von einander beabstandet sein können, als in einem weiter in dem Pressbereich gelegenen Abschnitt. Durch den im Aufnahmebereich 7 für den Tamponrohling 2 etwas größeren Abstand der Führungsklingen 4 voneinander wird gewährleistet, dass der Tamponrohling 2 auf einfache Weise in den zwischen den Führungsklingen 4 liegenden Raum eingeführt werden kann. Wie aus der Darstellung weiters ersichtlich ist, können die Führungsschienen 4 im Aufnahmebereich 7 voneinander weg gekrümmt sein, um den Eingangsbereich zu vergrößern und dadurch das Einführen des Tamponrohlings 2 zu erleichtern.

Der Tamponrohling 2 kann wie in Fig. 2 gezeigt, mittels eines beispielsweise stempel- oder rohrförmigen Einwerfers 10 in den Pressbereich geschoben werden. Anstelle des Einwerfers 10 kann der Tamponrohling 2 auch mittels einer hier nicht dargestellten Düse und unter Verwendung von Druckluft in den Pressbereich eingeblasen werden.

Grundsätzlich kann der Tamponrohling 2 auch auf andere Weise in den Pressbereich eingeführt werden, so könnten die Führungsklingen 4 beispielsweise als Fördermittel für den Tamponrohling 2 verwendet werden. In diesem Fall könnten die Führungsklingen 4 eine Förderbewegung in Richtung des Pressbereiches ausführen, um den Tamponrohling in seine in dem Pressbereich gelegene Pressposition zu transportieren. Auch wäre es möglich, dass die Führungsklingen 4 Fördermittel aufweisen, beispielsweise einen um die Führungsklingen 4 umlaufenden Riemen, der den Tamponrohling 2 entlang der Kanten 4a erfasst und diesen wie ein Förderband in den Pressbereich transportiert.

Eine andere Variante der erfindungsgemäßen Lösung wäre beispielsweise, dass die Führungsklingen 4 einen aus dem Pressbereich entnehmbaren und in diesen einführbaren Käfig zur Aufnahme des Tamponrohlings 2 bilden. Der von den Führungsklingen gebildete Käfig kann als Ganzes nach Aufnahme des Tamponrohlings 2 in die Presse 1 eingesetzt werden. Somit könnten die Führungsklingen 4 und der Tamponrohling 2 auch gemeinsam bzw. gleichzeitig in die Presse eingesetzt werden, wobei das Einführen des Tamponrohlings 2 zwischen die Führungsklingen 4 hierbei außerhalb der Presse 1 stattfinden kann.

Bei allen Ausführungsformen der Erfindung sind die Führungsklingen 4 jedoch so angeordnet, dass sie während des Pressvorganges, durch welchen der Tamponrohling 2 zu dem Tamponvorformling 2' verdichtet wird, ortsfest in der Presse 1 angeordnet sind und die Bewegung der Pressbacken 3 nicht einschränken.

In einem in dem Pressbereich gelegenen Abschnitt können die Führungsklingen 4 beispielsweise parallel zueinander verlaufen, wobei der Abstand der Führungsklingen 4 vorteilhafterweise so gewählt ist, dass Abschnitte der Führungsklingen 4 an der Oberfläche des Tamponrohlings 2 anliegen. Der Abstand der Führungsklingen 4 kann jedoch auch so gewählt sein, dass Abschnitte der Führungsklingen 4 sich in die Oberfläche des Tamponrohlings 2 eindrücken. Durch die in diesem Abschnitt erwähnten Maßnahmen kann eine gute Zentrierung des Tamponrohlings 2 in dem Pressbereich erzielt werden.

Gemäß einer Variante der Erfindung kann auch vorgesehen sein, dass der Abstand zwischen den Führungsklingen verstellt werden kann, dies kann beispielsweise dadurch erreicht werden, dass die Führungsklingen 4 an unterschiedlichen Stellen der Aufnahmen 6 des Befestigungsringes 5 befestigt werden können, sodass eine Führungsklinge 4 je nach Bedarf entweder näher oder entfernter dem Zentrum des Pressbereichs angeordnet werden kann.

Gemäß eine weiteren Ausführungsform der Erfindung kann der Abstand zwischen den Führungsklingen 4 auch während des laufenden Betriebes verändert werden. Dies kann beispielsweise dadurch realisiert werden, dass die Führungsklingen 4 in radialer Richtung verschiebbar in den Aufnahmen 6 gelagert sind.

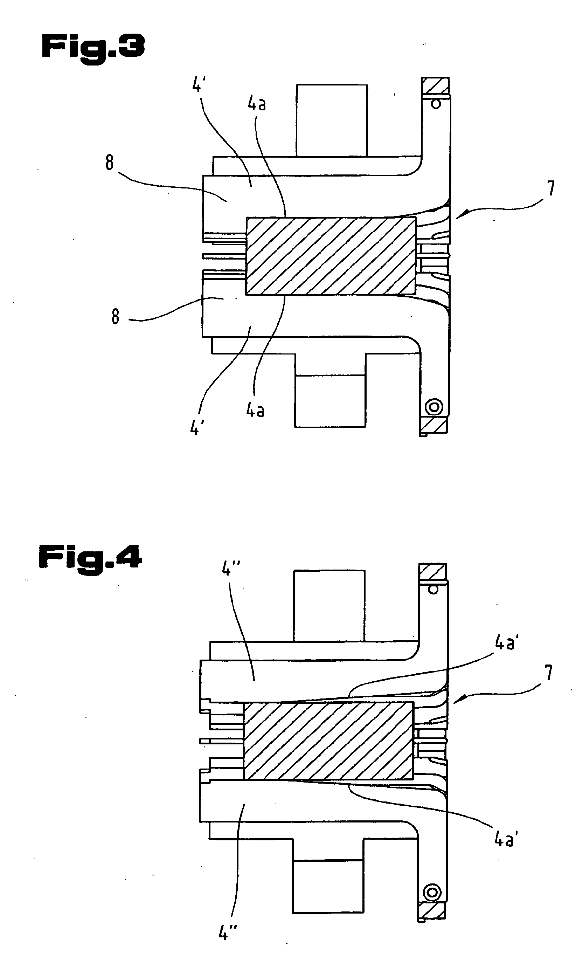

Wie in Fig. 3 dargestellt, können die Führungsklingen 4' abschnittsweise im Wesentlichen parallel zueinander verlaufen. D.h. die Kanten 4a verlaufen abschnittsweise im Wesentlichen parallel zueinander. Hierbei können die Führungsklingen 4' einen Anschlag 8 aufweisen, um die Bewegung des Tamponrohlings 2 in dem Pressbereich in axialer Richtung zu begrenzen und so eine exakte Definition der Lage des Tamponrohlings 2 in axialer Richtung zu ermöglichen.

Der Anschlag 8 kann hierbei durch in das Innere des Pressbereiches weisende Vorsprünge der einzelnen Führungsklingen 4' gebildet sein. Es ist jedoch nicht unbedingt erforderlich, dass alle Führungsklingen 4' Vorsprünge zur Bildung des Anschlages 8 aufweisen. So kann es auch ausreichend sein, wenn der Anschlag 8 von dem Vorsprung nur einer der Führungsklingen 4' gebildet wird.

Gemäß der in Fig. 4 dargestellten Variante der Erfindung können die Führungsklingen 4" an ihren zur Führung des Tamponrohlings 2 vorgesehenen Kanten 4a' so geformt sein, dass der Abstand zwischen den Kanten 4a' der Führungsklingen 4" von dem Aufnahmebereich weg abnimmt. Die Kanten 4a' der Führungsklingen 4" können hierbei konisch aufeinander zu laufen. Dadurch kommt es in einem in Bewegungsrichtung des Tamponrohlings 2 hinteren Bereich der Führungsklingen 4" zu einer eine weitere Bewegung in axialer Richtung verhindernden Klemmung des Tamponrohlings 2. Auf diese Weise ist sowohl eine exakte Positionierung des Tamponrohlings 2 in axialer als auch in radialer Richtung möglich.

Gemäß den Figur 5 können die Führungsklingen 4, wie bereits oben erwähnt, an der Ausstossdüse 9 angeordnet sein. Wie aus den Fig. 6 bis 8 weiters ersichtlich, können die Führungsklingen 4 an einem um eine Öffnung 12 der Ausstossdüse 9 verlaufenden Ring 11 angeordnet sein. Die Führungsklingen 4 können hierbei mit der Ausstossdüse 9 einstückig ausgebildet oder mit dieser verbunden sein. So können die Führungsklingen 4 mit der Ausstossdüse 9 beispielsweise verklebt, verschweißt, verlötet oder verschraubt bzw. auf eine beliebige andere Art mit der Ausstossdüse 9 verbunden sein. Sowohl die Ausstossdüse 9 als auch die Führungsklingen 4 können hierbei aus Kunststoff oder Metall gefertigt sein. Auch in der in den Figuren 5 bis 8 gezeigten Ausführungsform sind die Führungsklingen 4 ortsfest in der Presse 1 montiert.

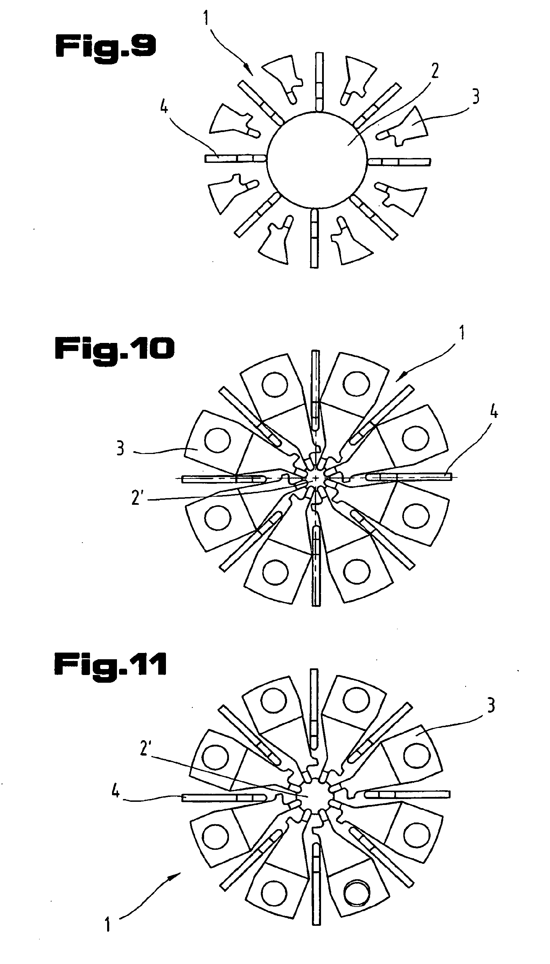

Wie aus Fig. 9 ersichtlich ist, sind die Pressbacken 3 während des Einführens des Tamponrohlings 2 in die Presse 1 geöffnet, wobei während dieses Vorganges nur die Führungsklingen 4 an dem Tamponrohling 2 anliegen. Nach Positionierung des Tamponrohlings 2 in dem Pressbereich werden die Pressbacken 3 auf den Tamponrohling 2 zu bewegt.

Aus Fig. 10 ist ersichtlich, dass dass die Pressbacken 3 sich während des Pressens radial in Richtung des Tamponrohlings 2 bewegen und diesen komprimieren, während die ortsfesten Führungsklingen 4 an ihren ursprünglichen Positionen verbleiben und während des Pressvorganges den Kontakt zu dem Tamponrohling 2' verlieren. Durch die ortsfeste Anordnung der Führungsklingen 4 kann verhindert werden, dass die Bewegung der Pressbacken 3 während des Pressvorganges durch die Führungsklingen 4 behindert wird.

Fig. 11 zeigt die Presse 1 in einer Position, in welcher der durch Pressen bzw. Verdichten des Tamponrohlings 2 hergestellte Tamponvorformling 2' aus der Presse 1 ausgestoßen wird. Hierbei sind die Pressbacken 3 von dem Tamponvorformling 2' etwas zurückgefahrenen. Wie aus der Darstellung ersichtlich ist, liegen die Führungsklingen 4 gemäß der bevorzugten Ausführungsform hierbei nicht an dem Tamponvorformling 2' an.

An dieser Stelle sei aus Gründen der Vollständigkeit erwähnt, dass obwohl die dargestellten Pressbacken 3 Eindringungssegmente zum Einprägen von Rillen aufweisen, auch Pressbacken beliebiger anderer Form, welche keine derartigen Eindringungssegmente aufweisen, zur Realisierung der Erfindung verwendet werden können und die Erfindung nicht auf die dargestellten Pressbacken 3 beschränkt ist.

### Bezugszeichenaufstellung

- 1: Presse
- 2: Tamponrohling
- 2': Tamponvorformling
- 3: Pressbacken
- 4: Führungsklingen
- 4a, 4a': Kanten der Führungsklingen
- 5: Befestigungsring

- 6: Aufnahmen
- 7: Aufnahmebereich
- 8: Anschlag
- 9: Ausstossdüse
- 10: Einwerfer

- 11: Ring der Ausstossdüse
- 12: Öffnung der Ausstossdüse

## Patentansprüche

1. Presse (1) zur Herstellung eines Tamponvorformlings (2') oder eines Tampons durch radiales Pressen eines aus einem absorbierenden Material hergestellten Tamponrohlings (2) mittels Pressbacken (3), welche um einen Pressbereich angeordnet sind, **dadurch gekennzeichnet, dass** die Presse (1) ortsfest in der Presse (1) montierte Führungsklingen (4, 4', 4") zur Zentrierung des Tamponrohlings (2) in radialer und/oder axialer Richtung in dem Pressbereich aufweist.

2. Presse nach Anspruch 1, **dadurch gekennzeichnet, dass** die Führungsklingen (4, 4', 4") radial um den Pressbereich angeordnet sind.

3. Presse nach Anspruch 2, **dadurch gekennzeichnet, dass** jede Führungsklinge (4, 4', 4") zwischen zwei benachbarten Pressbacken (3) angeordnet ist.

4. Presse nach einem der Ansprüche 1 bis 3, **dadurch gekennzeichnet, dass** die Führungsklingen (4") in Einführrichtung des Tamponrohlings (2) in den Pressbereich aufeinander zulaufend angeordnet und/oder ausgebildet sind.

5. Presse nach einem der Ansprüche 1 bis 4, **dadurch gekennzeichnet, dass** zumindest drei Führungsklingen (4, 4', 4") vorgesehen sind.

6. Presse nach einem der Ansprüche 1 bis 5, **dadurch gekennzeichnet, dass** die Führungsklingen (4, 4', 4") in einem zur Aufnahme des Tamponrohlings (2) vorgesehenen Aufnahmebereich (7) weiter von einander beabstandet sind als in einem von dem Aufnahmebereich (7) entfernten Abschnitt.

7. Presse nach einem der Ansprüche 1 bis 6, **dadurch gekennzeichnet, dass** zumindest eine Führungsklingen (4') an ihrem in dem Pressbereich gelegenen Ende einen Anschlag (8) für den Tamponrohling (2) aufweist.

8. Presse nach einem der Ansprüche 1 bis 7, **dadurch gekennzeichnet, dass** die Führungsklingen (4, 4', 4") federelastisch ausgebildet sind.

9. Presse nach einem der Ansprüche 1 bis 8, **dadurch gekennzeichnet, dass** die Führungsklingen (4, 4', 4") plattenförmig ausgebildet sind, wobei je eine dem das Inneren der Presse (1) zugewandte Kante (4a, 4a') der plattenförmigen Führungsklingen (4, 4', 4") zur Führung des Tamponrohlings (2) vorgesehen ist.

10. Presse nach einem der Ansprüche 1 bis 9, **dadurch gekennzeichnet, dass** die Führungsklingen (4, 4', 4") an einem ringförmigen Befestigungselement (5) angeordnet und von diesem an ihrer Position gehalten sind.

11. Presse nach einem der Ansprüche 1 bis 9, **dadurch gekennzeichnet, dass** die Führungsklingen (4, 4', 4") an einer Ausstossdüse (9) der Presse angeordnet sind.

12. Verfahren zur Herstellung eines Tamponvorformlings (2') durch radiales Pressen eines aus einem absorbierenden Material hergestellten Tamponrohlings (2) mittels Pressbacken (3), welche um einen Pressbereich einer Presse (1) angeordnet sind, **dadurch gekennzeichnet, dass** der Tamponrohling (2) während des Einführens in den Pressbereich mittels ortsfest in der Presse (1) montierter Führungsklingen (4) in radialer und/oder axialer Richtung in dem Pressbereich zentriert wird.

## Claims

1. Press (1) for producing a tampon preform (2') or a tampon by radially pressing a tampon blank (2) made of an absorbent material by means of compression jaws (3), which are arranged around a pressing region, **characterized in that** the press (1) comprises guide blades (4, 4', 4"), which are fixed in the press (1), for centering the tampon blank (2) in radial and/or axial direction in the pressing region.

2. Press according to claim 1, **characterized in that** the guide blades (4, 4', 4") are arranged radially around the pressing region.

3. Press according to claim 2, **characterized in that** each guide blade (4, 4', 4") is arranged between two adjacent compression jaws (3).

4. Press according to one of the claims 1 to 3, **characterized in that** the guide blades (4") are arranged and/or embodied to converge to one another in direction of insertion of the tampon blank (2) into the pressing region.

5. Press according to one of the claims 1 to 4, **characterized in that** at least three guide blades (4, 4', 4") are provided.

6. Press according to one of the claims 1 to 5, **characterized in that** the guide blades (4, 4', 4") in the collector region (7) provided for collecting the tampon blank (2) are spaced apart from one another more widely than in a section further away from the collecting region (7).

7. Press according to one of the claims 1 to 6, **characterized in that** at least one guide blade (4') comprises a stopper (8) for the tampon blank (2) at its ending situated in the pressing region.

8. Press according to one of the claims 1 to 7, **characterized in that** the guide blades (4, 4', 4") are embodied springy.

9. Press according to one of the claims 1 to 8, **characterized in that** the guide blades (4, 4', 4") are embodied to be plate-shaped, with one respective edge (4a, 4a') of the plate-shaped guide blades (4, 4', 4") facing the interior of the pressing region being provided for the guidance of the tampon blank (2).

10. Press according to one of the claims 1 to 9, **characterized in that** the guide blades (4, 4', 4") are arranged at an annular mounting element (5) and held in their position by the latter.

11. Press according to one of the claims 1 to 9, **characterized in that** the guide blades (4, 4', 4') are arranged at an outlet nozzle (9) of the press.

12. Method for producing a tampon preform (2') by radially pressing a tampon blank (2) made of an absorbent material by means of compression jaws (3), which are arranged around the pressing region of a press (1), **characterized in that** the tampon blank (2), during the insertion into the pressing region, is centered in radial and/or axial direction in the pressing section by means of guide blades (4) which are fixed in the press (1).

## Revendications

1. Presse (1) pour réaliser une préforme de tampon (2') ou un tampon par pressage radial d'une ébauche de tampon (2) réalisée dans un matériau absorbant au moyen de mâchoires de pressage (3) qui sont agencées autour d'une zone de pressage, **caractérisée en ce que** la presse (1) présente des lames de guidage (4, 4', 4") montées de façon stationnaire dans la presse (1) pour centrer l'ébauche de tampon (2) dans la direction radiale et/ou axiale dans la zone de pressage.

2. Presse selon la revendication 1, **caractérisée en ce que** les lames de guidage (4, 4', 4") sont agencées radialement autour de la zone de pressage.

3. Presse selon la revendication 2, **caractérisée en ce que** chaque lame de guidage (4, 4', 4") est agencée entre deux mâchoires de pressage (3) adjacentes.

4. Presse selon l'une des revendications 1 à 3, **caractérisée en ce que** les lames de guidage (4") sont agencées et/ou réalisées de façon à arriver l'une sur l'autre dans la zone de pressage dans la direction d'insertion de l'ébauche de tampon (2).

5. Presse selon l'une des revendications 1 à 4, **caractérisée en ce qu'**au moins trois lames de guidage (4, 4', 4") sont prévues.

6. Presse selon l'une des revendications 1 à 5, **caractérisée en ce que** les lames de guidage (4, 4', 4") sont situées à une plus grande distance l'une de l'autre dans une zone de réception (7) prévue pour recevoir l'ébauche de tampon (2) que dans une section éloignée de la zone de réception (7).

7. Presse selon l'une des revendications 1 à 6, **caractérisée en ce qu'**au moins une lame de guidage (4') présente une butée (8) pour l'ébauche de tampon (2) au niveau de son extrémité située dans la zone de pressage.

8. Presse selon l'une des revendications 1 à 7, **caractérisée en ce que** les lames de guidage (4, 4', 4") sont réalisées de façon flexible.

9. Presse selon l'une des revendications 1 à 8, **caractérisée en ce que** les lames de guidage (4, 4', 4") sont réalisées en forme de plaques, étant entendu que pour chacune, l'arête (4a, 4a') de la lame de guidage en forme de plaque (4, 4', 4") dirigée vers l'intérieur de la presse (1) est prévue pour guider l'ébauche de tampon (2).

10. Presse selon l'une des revendications 1 à 9, **caractérisée en ce que** les lames de guidage (4, 4', 4") sont agencées sur un élément de fixation annulaire (5) et maintenues dans leur position par celui-ci.

11. Presse selon l'une des revendications 1 à 9, **caractérisée en ce que** les lames de guidage (4, 4', 4") sont agencées sur une buse d'éjection (9) de la presse.

12. Procédé de réalisation d'une préforme de tampon (2') par pressage radial d'une ébauche de tampon (2) réalisée dans un matériau absorbant au moyen de mâchoires de pressage (3) qui sont agencées autour d'une zone de pressage d'une presse (1), **caractérisé en ce que** l'ébauche de tampon (2) est centré dans la direction radiale et/ou axiale dans la zone de pressage pendant son insertion dans la zone de pressage au moyen de lames de guidage (4) montées de façon stationnaire dans la presse (1).
